# FASCICULE DE BREVET EUROPEEN

(11) **EP 0 425 381 B1**
(45) Date de publication et mention de la délivrance du brevet: **22.02.1995**
(21) Numéro de dépôt: 90403006.1
(22) Date de dépôt: 25.10.1990
(51) Int. Cl.: G01N 15/12, G01N 15/14

(54) **Appareil pour le comptage et la détermination d'au moins une sous-population leucocytaire**
Vorrichtung zum Zählen und zur Bestimmung mindestens einer Leukozytensubpopulation
Apparatus for counting and determination of at least one leucocyte-subpopulation

(30) Priorité: 27.10.1989 FR 8914120
(43) Date de publication de la demande: 02.05.1991
(73) Titulaire: ABX , Société Anonyme dite, F-34184 Montpellier Cédex 4 (FR)
(72) Inventeur: Lefevre, Didier, F-78200 Mantes la Ville (FR); Champseix, Henri, F-78360 Montesson (FR); Champseix, Serge, F-78130 Les Mureaux (FR)
(74) Mandataire: Lhuillier, René

(56) Documents cités:
- EP-A- 0 121 261
- EP-A- 0 165 868
- EP-A- 0 310 504
- DE-A- 3 310 551
- US-A- 3 710 933

## Description

L'invention a pour objet le comptage et l'analyse d'au moins une sous-population leucocytaire et concerne plus précisément un appareil assurant cette identification par mesurage optique et électronique.

Indépendament des méthodes manuelles de comptage et d'analyse d'une sous-population leucocytaire qui consistent à visualiser au microscope des échantillons de sang étalés sur une lame, et préalablement colorés, il existe des méthodes automatiques beaucoup plus performantes et précises qui permettent de différencier simultanément plusieurs types de populations sanguines. On connait essentiellement deux types de méthodes d'analyse : les méthodes d'analyse de taille par résistivité et les méthodes d'analyse optique.

L'analyse et le comptage automatique par résistivité sont basés sur le principe de passage d'une cellule dans un champ électrique où est entretenu un courant constant. La résistance qu'oppose cette cellule dans le champ provoque une augmentation de la tension nécessaire à la constance du courant selon la loi d'Ohm. L'impulsion en tension, générée par le passage de cette cellule, est proportionnelle à la résistance opposée, donc à son volume sans considération de forme. Afin de déterminer une sous-population leucocytaire par ce principe il est nécessaire de traiter préalablement l'échantillon de sang au moyen d'agents cytochimiques spécifiques permettant la destruction partielle des cellules n'étant pas à considérer ceci afin d'obtenir une discrimination de taille aussi précise que le traitement est spécifique.

La méthode de différenciation la plus communément utilisée avec ce principe est la méthode dite "Screening Leucocytaire", permettant d'obtenir une approche de la formule sanguine sur trois populations : les Lymphocytes, les cellules Mononucléées et les Granulocytes.

L'essentiel de l'analyse repose sur l'utilisation d'une réactif lytique à action différentielle. Par le biais de ce réactif les membranes des leucocytes laissent fuir le contenu du cytoplasme. Les cellules n'ayant pas de granules contenus dans leur cytoplasme se retrouvent donc, avec la membrane cytoplasmique enrobant leur noyau ; les granulocytes, eux, conservent une partie du cytosol, leurs granules empêchant partiellement sa fuite.

L'inconvénient majeur de cette méthode réside dans le fait que les leucocytes ne sont différenciés que par leur taille finale et que l'action de la lyse sur certaines cellules, notamment les éosinophiles et les basophiles, n'est pas maîtrisée totalement, ceci provoquant fréquement un chevauchement voir une superposition des populations qui rendent la différenciation impossible où trés hasardeuse.

D'un autre coté l'analyse et le comptage d'une sous-population leucocytaire par les méthodes optiques utilisent soit le principe de mesure de diffraction optique, soit le principe de mesure de densité optique d'une cellule, soit une combinaison des deux.

Dans le premier cas, une cellule traversant un rayon lumineux engendre une diffraction de la lumière incidente, dont l'intensité à différents angles est fonction de la taille de la cellule et de la quantité de lumière absorbée par celle-ci. Un collecteur optique ayant en son centre un disque à fond noir est placé dans l'alignement du trajet optique. La lumière transmise est stoppée par le disque tandis que la lumière diffractée est collectée sur un capteur photosensible dont la réponse est proportionnelle à la diffraction de la cellule.

Dans le second cas, une cellule traversant un rayon lumineux engendre une absorbance de la lumière incidente. La lumière transmise est filtrée à la longueur d'onde correspondant à la coloration de la cellule, puis est collectée sur un capteur photosensible dont la réponse est proportionnelle à la lumière absorbée à la longeuer d'onde spécifiée.

La diffraction cellulaire étant liée à l'absorbance de la cellule ainsi qu'à sa forme, les mesures de volume restent aléatoires, notamment pour ce qui est des leucocytes et il est nécessaire de rendre artificiellement sphériques les cellules à mesurer.On note aussi qu'une cellule de grande taille très colorée sera vue plus petite que sa taille réelle.

Des combinaisons de ces principes sont utilisées afin d'obtenir pour une même cellule, des valeurs de diffraction et d'intensité de coloration ou des valeurs de diffraction à des longueurs d'onde différentes, ou encore, des valeurs de diffraction à des angles différents. Par le truchement de cytochimies spécifiques il est obtenu une discrimination cellulaire.

Les appareillages utilisant ces principes souffrent d'une grande sensibilité de l'alignement optique rendant les mesures de diffraction d'une stabilité relative. De nombreux facteurs fragilisent également la mesure comme, notamment, l'encrassement de la cuve de lecture, la sensibilité des parties optiques aux poussières atmosphériques et à la température et l'hygromètrie du local. En outre, la haute technicité nécessaire à un ensemble optique performant rend le coût de l'appareil peu attractif.

L'invention a donc pour objet un appareil qui évite les inconvénients inhérents aux appareils mettant en application les méthodes précitées et qui permet l'identification, le comptage et/ou l'analyse d'au moins une sous-population leucocytaire et en particulier les polynucléaires éosinophiles.

Ainsi l'objet de la présente invention réside en un appareil pour le comptage et la détermination d'au moins une sous-population leucocytaire utilisant au moins en partie une méthode d'analyse et de comptage par résistivité basée sur le principe de passage d'une cellule dans un champ électrique où est entretenu un courant constant fournissant un signal interprété en vue d'obtenir une information sur le volume de la cellule traversant ce champ et utilisant au moins en partie une méthode optique consistant à faire passer une cellule dans un rayon lumineux et à recueillir dans un collecteur optique la lumière absorbée par la cellule pour fournir un signal interprété en vue d'obtenir une information sur l'absorbance de la cellule, lequel appareil comportant essentiellement un boîtier pour l'injection, à l'intérieur d'une cuve de mesure, du flux d'échantillon à analyser provenant d'un circuit d'alimentation en amont du boîtier d'injection, laquelle cuve est traversée par un faisceau lumineux recueilli par un capteur et des unités de recueil et de traitement des signaux fournis par le capteur optique et par les électrodes servant à engendrer le champ électrique étant prévues en aval du boîtier d'injection, caractérisé en ce que le flux d'échantillon est manchonné hydrodynamiquement à l'intérieur de la cuve par deux liquides de manchonnage sous pression injectés dans la cuve par deux conduits d'alimentation séparés, disposés en amont de la cuve, le second manchonnage enveloppant le premier flux manchonné, et en ce que les deux électrodes pour les mesures de résistivité de la sous-population leucocytaire sont des éléments constitués d'une part, d'une anode formée par l'embout du conduit d'évacuation du flux manchonné de solution sortant de la cuve, et d'autre part, d'une cathode formée par une buse interne d'injection de la solution à analyser.

Le document EP-A-0 165 868 divulgue un appareil tel que décrit dans le préambule de la revendication 1.

Selon une caractéristique de l'invention, une buse externe d'injection est montée à l'intérieur du boitier et se termine par un orifice calibré qui fait communiquer la chambre intérieure de ladite buse avec la cuve, pour l'émission d'un premier liquide de manchonnage.

Avantageusement, une chambre annulaire est ménagée entre une partie conique de la buse externe d'injection et une face interne du boitier ; elle communique avec la cuve de mesure et elle est alimentée en fluide sous pression par au moins un conduit.

Selon une autre caractéristique particulière de l'invention, une buse interne d'injection est montée à l'intérieur de la buse externe et porte un orifice d'injection de fluide sous pression provenant d'un conduit qui débouche dans une chambre intérieure à ladite buse externe au droit de l'orifice calibré.

L'invention prévoit également des parois de verre de part et d'autre de la cuve de mesure, et qui sont encadrées par, d'un coté une lampe et de l'autre par un capteur qui reçoit le faisceau émis par la lampe et focalisé par des éléments optiques, qui a traversé le flux manchonné de la solution à analyser, passant dans la cuve.

L'interprétation des résultats fournis par l'appareil est avantageusement obtenue selon l'invention par le fait que les informations provenant des mesures de résistivité sont restituées sur une courbe de répartition des tailles permettant de localiser entre un seuil bas et un seuil haut la population principale de leucocytes, en séparant les stromas et plaquettes ainsi que les particules de trés grande taille.

De même les informations provenant des mesures optiques sont restituées par une courbe de répartition des absorbances permettant de localiser entre un seuil bas et un seuil haut l'absorbance des leucocytes en séparant les populations de cellules peu absorbantes ainsi que les populations de cellules à absorption intense.

De plus il apparait également avantageux que les informations provenant des mesures de résistivité et des mesures optiques puissent être resituées dans une représentation graphique en matrice, permettant de visualiser des contours représentatifs de la répartition des populations.

D'autres caractéristiques et avantages de l'invention serant mieux perçus à la lecute de la description qui va suivre qui fait référence aux dessins annexés qui représentent
Figure 1 une vue en coupe de boitier d'injection.
Figure 2 une vue schématique des circuits de préparation et de traitement associés à l'appareil.
Figure 3 une courbe de répartition des tailles des leucocytes analysés.
Figure 4 une courbe de répartition des absorbances des leucocytes analysés.
Figure 5 une représentation graphique matricielle des contours représentatifs de la répartition des populations.

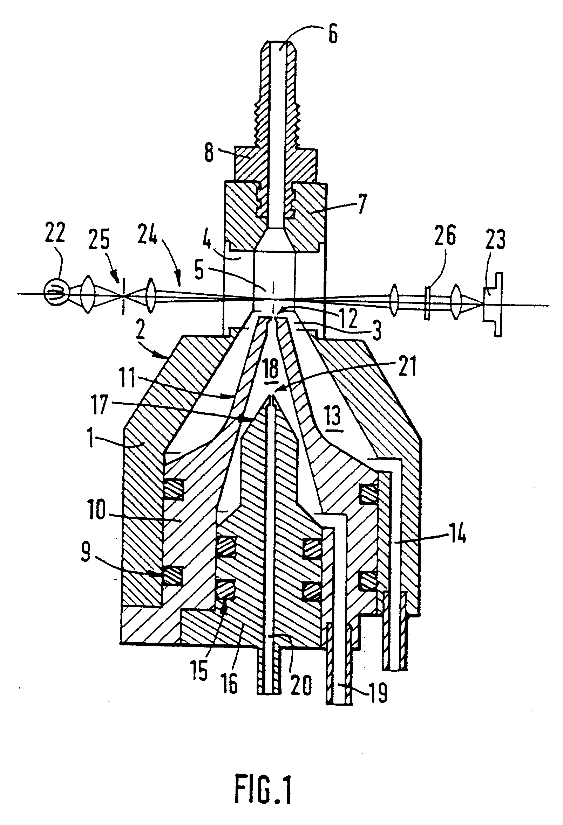

L'appareil représenté à la figure 1 comporte un boitier d'injection 1 de forme générale cylindrique dont la partie supérieure présente un profil conique 2. L'orifice 3 du boitier 1 est coiffé sur au moins deux faces opposées, de parois de verre 4 entre lesquelles est disposée une cuve de mesure 5 communiquant par sa base avec le boitier d'injection et débouchant à sa partie supérieure dans un conduit d'évacuation 6. Un bouchon 7, appliqué sur les parois de verre 4, maintient la cuve 5 et sert également de support à l'embout 8 du conduit d'évacuation.

A l'intérieur du boitier d'injection 1 est montée de façon étanche grâce à des joints toriques 9 une buse externe d'injection 10 dont l'extrémité supérieure 11 de forme conique débouche à la base de la cuve de mesure 5. Elle se termine par un orifice calibré 12, par exemple une pierre à trou qui fait communiquer la chambre intérieure 18 de ladite buse avec la cuve 5. On notera qu'entre la face interne de la partie conique 2 du boitier d'injection, et la face externe de l'extrémité conique 11 de la buse 10, se trouve ménagée une chambre annulaire 13 qui communique également avec la cuve de mesure. Cette chambre 13 est alimentée en fluide sous pression par un conduit 14.

A l'intérieur de la buse externe d'injection 10 est montée de façon étanche grâce à des joints toriques 15 une buse interne d'injection 16 dont l'extrémité supérieure 17, également de forme conique s'étend dans la chambre intérieure 18 de la buse externe 10, au dessous de l'orifice calibré 12. La chambre 18 est elle-même alimentée en fluide sous pression par un conduit 19. De même la buse interne est traversée par un conduit 20 permettant l'injection de fluide sous pression par l'orifice 21 de ladite buse, au droit de l'orifice calibré 12. Les deux parois de verre 4 sont encadrées par, d'un coté une lampe 22 et de l'autre un capteur 23 de telle sorte que le faisceau lumineux 24 émis par la lampe et focalisé par un projecteur optique approprié 25 traverse ces parois et la cuve de mesure 5 et soient recueillis par le capteur après passage au travers d'un autre système optique 26.

La solution de leucocytes à analyser est injectée par le conduit central 20 et s'échappe par l'orifice 21 dans la chambre 18. Simultanément on injecte par le conduit 19 un liquide sous pression dit liquide de manchonnage réalisant un gainage hydrodynamique de la solution dans l'orifice calibré 12. Le flux manchonné provenant de ce premier manchonnage traverse donc l'orifice calibré 12, et se trouve alors soumis à un deuxième manchonnage réalisé par injection de liquide sous pression par le conduit 14 et orienté à l'intérieur de la chambre annulaire 13. Ce manchonnage débouche par l'orifice 3 dans la cuve de mesure 5, traversée, perpendiculairement au flux, d'un faisceau lumineux 24, concentré et focalisé sur le flux de solution de leucocytes. La circulation des flux dans l'ensemble de mesure se fait du bas vers le haut, selon un axe vertical, évitant ainsi la stagnation d'éventuelles bulles. Le fait d'utiliser ainsi un double manchonnage présente un certain nombre d'avantages.

Le premier manchonnage réalise un centrage parfait du flux de cellules dans l'orifice de comptage et interdit, de par la finesse du flux réalisé et du ratio de dilution, le passage coïncidant de plusieurs cellules. Les phénomènes de déformation des cellules dus aux effets de bord ainsi que de rebond des cellules dans la zone de sensibilité de l'orifice de comptage, sont également éliminés.

Le second manchonnage enveloppe le flux sortant de l'orifice et le maintient concentrique et stabilisé pendant tout le trajet dans la cuve optique, autorisant ainsi une ou plusieurs lectures à différents angles et à différents niveaux.

Ce second manchonnage permet d'utiliser une cuve de lecture optique de large passage intérieur éliminant les turbulences des effets de bord et le risque d'encrassement qui auraient pour effets de rendre le flux instable et la qualité optique médiocre.

Le fait d'utiliser une cuve de larges dimensions élimine également la vitesse du flux sur ces parois; cette vitesse étant faible, l'érosion des parois de verre,- que l'on peut constater à long terme sur les cuves optiques capillaires dans lesquelles la vitesse du manchon doit être égale à la vitesse du flux - est diminuée.

Le cumul de ces caractéristiques ajouté au fait que le positionnement du flux est réalisé par le positionnement de l'orifice de comptage, rend l'alignement du faisceau optique sur le flux extrêmement stable. Un démontage de la cuve ne justifie nullement un réglage optique après son remontage.

Le comptage et la détection du volume sont assurés par des mesures de résistivité. A cet effet on applique un courant aux bornes de deux électrodes situées de part et d'autre de l'orifice 12, à savoir une anode constituée par l'embout 8 du conduit d'évacuation et une cathode constituée de la buse interne d'injection 16. Le comptage des leucocytes est réalisé lors du passage de la solution dans l'orifice calibré 12 ; chaque cellule passant dans l'orifice provoque une augmentation de la résistivité du milieu situé entre les électrodes (8, 16), créant ainsi une impulsion en tension proportionnelle au volume du leucocyte.

La détection optique c'est à dire la détermination de l'intensité de l'absorpiton des leucocytes est mesurée au moyen du faisceau lumineux 24 traversant la cuve 5 perpendiculairement au flux à analyser. Le faisceau lumineux est réalisé au moyen de la lampe 22, dont l'énergie lumineuse passant au travers d'un filtre de longueur d'onde correspondant à l'absorption de la cellule, puis d'une fenêtre est concentrée sur un diaphragme derrière lequel est placé l'ensemble projecteur optique 25, focalisé sur le flux de solution de leucocytes. L'image de la fenêtre lumineuse traversée par le flux de solution, passe par un collimateur puis est projetée par l'autre système optique collecteur 26 sur une photodiode 23 aux bornes de laquelle est connecté un montage amplificateur.

Chaque leucocyte traversant le faisceau lumineux, provoque une réduction de l'intensité lumineuse mesurée sur la photodiode, proportionnelle à l'intensité de son absorption. Cela se traduit par une impulsion électrique aux bornes de l'amplificateur, dont l'amplitude est elle-même, proportionnelle à la densité optique du leucocyte.

L'orifice calibré 12 se trouve, comme on le remarque à la figure 1, légèrement à l'écart du faisceau lumineux 24, traversant la cuve.

On notera que cette distance séparant l'orifice 12 du point de focalisation de mesure optique engendre un décalage temporel des impulsions résistives et optiques.

La constance de ce décalage est contrôlée et permet de mettre en évidence la moindre chute de performance de la fluidique.

Les micro-bulles sont naturellement éliminées du comptage par la résistance aux flux vertical qu'oppose la bulle d'air, engendrant ainsi un décalage dans les comptages résistifs et optiques supérieur au décalage standard engendré par une cellule.

Pour être prise en compte, une cellule sanguine doit, préalablement à la mesure optique, être mesurée de manière résistive par son passage dans l'orifice calibré 12. Ainsi les éventuelles particules contenues dans le manchon liquide injecté après l'orifice de comptage ne peuvent être prises en compte, n'ayant pas généré d'impulsion résistive.

Avant de procéder aux injections de sang dans l'appraeil décrit plus haut, il convient de préparer l'échantillon de sang à analyser de manière à obtenir une solution contenant principalement les leucocytes dans leur forme la plus naturelle possible Si nécessaire les cellules peuvent être spécifiquement colorées par un moyen cytochimique déterminé .

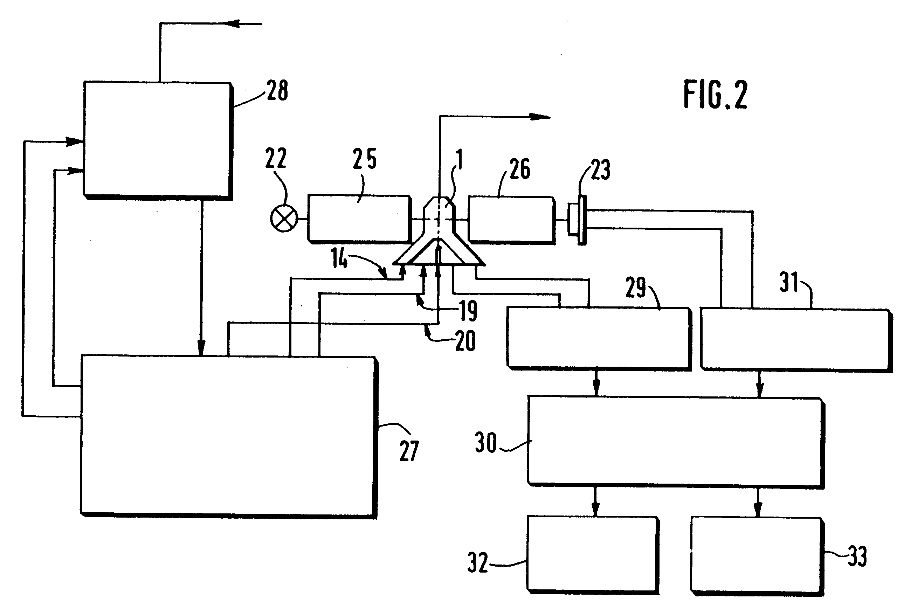

On a représenté à la figure 2 les circuits de traitement associés à l'appareil de la figure 1. On a schématisé le boitier 1 et son ensemble optique associé constitué de la lampe 22, du projecteur optique 25, du système optique collecteur 26 et la photodiode de réception 23. Le sang admis dans le boitier 1 par conduit 20, ainsi que les fluides de manchonnage admis par les conduits 14 et 19 subissent une préparation et proviennent d'une ensemble de distribution hydraulique 27, lui-même alimenté par un échantillon de sang provenant d'une unité 28 de mélange et de chauffage. Les électrodes localisées dans le boitier 1 fournissent des signaux proportionnels au volume des leucocytes reçus dans un circuit analogique 29 de mesure de résistivité, signaux convertis en valeur numérique dans une unité 30 de traitement numérique des informations. La photodiode 23, de son coté, fournit des signaux proportionnels à la densité optique des leucocytes reçus dans un circuit analogique 31 de mesure d'absorbance, signaux également traités dans l'unité 30 qui fournit ensuite des résultats graphiques 32 ou numériques 33. L'ensemble des signaux d'un même échantillon est traité au moyen d'un calculateur afin de déterminer le nombre de leucocytes comptés dans un temps préétabli ainsi que des valeurs relatives de volume et de densité optique pour chacun d'eax.

Quand la solution de sang traité passe dans l'appareil de mesure 1, chaque cellule provoque alternativement une impulsion proportionnelle à sa taille lorsqu'elle passe dans l'orifice calibré 12, puis une impulsion proportionnelle à son absorbance lors de son passage dans le faisceau lumineux 24. Pour une même cellule, une valeur de volume et une valeur d'absorbance sont mémorisées et les résultats totaux des volumes et des absorbances sont répartis selon un histogramme.

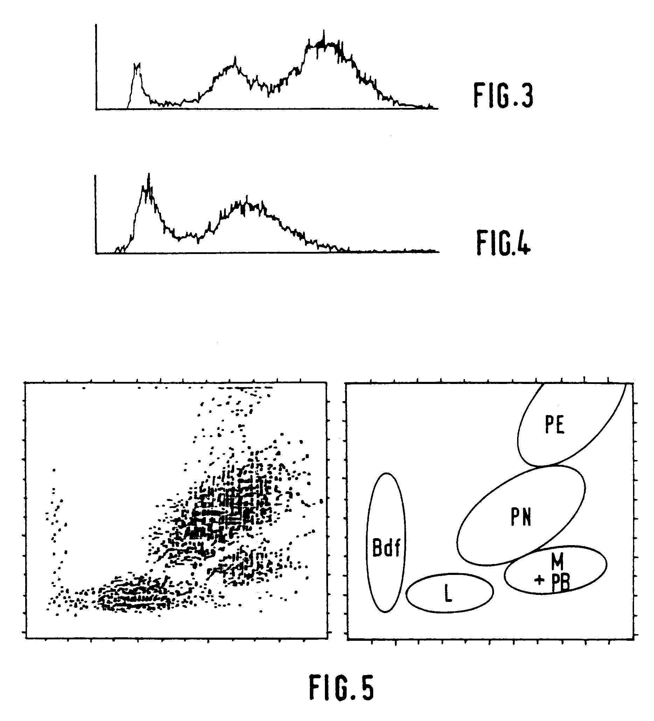

Sur une courbe de répartition des tailles présentée à titre d'exemple à la figure 3, avec en ordonnée la représentation quantitative et en abscisse le volume, on note la présence de trois populations distinctes. La population la plus à gauche, donc de particules de petites tailles, est considérée comme étant le bruit de fond composé principalement des stromas, résultants de l'hémolyse, et des plaquettes.

Les deux autres populations de droite sont considérées comme étant la totalité des leucocytes de l'échantillon analysé. Sur une courbe de répartition des absorbances présentée aussi à titre d'exemple à la figure 4, on obtient une première population à gauche constituée de cellules peu ou faiblement absorbantes, une seconde population centrale constituée de cellules à absorbance moyenne, et une troisième population plus à droite constituée de cellules de forte absorbance.

Sur une représentation graphique matricielle presentée également à titre d'exemple à la figure 5, on observe la répartition des populations en taille selon l'axe des X et en absorbance selon l'axe des Y. Cette représentation permet l'étude des leucocytes par un logiciel de taxonomie, le positionnement de seuils permettant la séparation d'au moins une population de leucocytes, Bdf représentant les bruits de fond (stomas + plaquettes) :
L : Lymphocytes
M : Monocytes
PN : Polynucléaires neutrophiles
PE : Polynucléaires éosinophiles
PB : Polynucléaires basophiles.

L'invention ne se limite pas aux modes de réalisation décrits ni à ce type d'analyses. Notamment on peut envisager, en remplaçant le filtre optique par une roue à filtres, de litre des colorations multiples autorisant l'analyse d'autres types cellulaires.

De plus, on a parlé précédemment d'une cuve de mesure optique .Selon une variante de réalisation il serait possible de mesurer la diffraction lumineuse d'une cellule traversant cette cuve. Un ou plusieurs capteurs étant disposés focalisés sur la cuve, dans l'alignement d'un rayon lumineux traversant celle-ci. Les informations recueillies, selon que l'on mesure la diffraction totale ou les diffractions à plusieurs angles, permettent la détermination soit de la taille relative de la cellule, soit, dans le cas deu deux mesures de diffraction à deux angles différents, de la taille et de la densité optique relative.

En variante, il serait possible également de réaliser une mesure de cytofluorescence en positionnant un ensemble capteur focalisé sur la cuve de mesure, à 90 degrés d'un rayon laser la traversant. L'ensemble de préparation de l'échantillon devant être adapté afin d'obtenir la fluorescence cellulaire recherchée. Les mesures ainsi obtenues permettent d'évaluer le volume de la cellule par résistivité, et sa fluorescence.

En variante encore, et toujours selon le principe même de l'ensemble de mesure, le courant appliqué aux électrodes et circulant dans l'orifice de comptage, peut être un courant à haute fréquence permettant d'obtenir, selon la forme des impulsions recueillies, une identification sur la composition interne de la cellule, soit la taille relative du noyau, plus dense par rapport à l'enveloppe externe (cytoplasme) de la cellule.

La combinaison des variantes exposées ci-dessus permet d'obtenir pour un flux de cellules traversant la cuve de mesure, à la fois le volume global de chaque cellule, associé à sa densité optique et/ou sa fluorescence, ainsi que des informations sur sa composition interne, à savoir la taille relative de son noyau et/ou sa forme relative ou sa régularité.

## Revendications

1. Appareil pour le comptage et la détermination d'au moins une sous-population leucocytaire utilisant au moins en partie une méthode d'analyse et de comptage par résistivité basée sur le principe de passage d'une cellule dans un champ électrique où est entretenu un courant constant fournissant un signal interprété en vue d'obtenir une information sur le volume de la cellule traversant ce champ et utilisant au moins en partie une méthode optique consistant à faire passer une cellule dans un rayon lumineux et à recueillir dans un col lecteur optique la lumière absorbée par la cellule pour fournir un signal interprété en vue d'obtenir une information sur l'absorbance de la cellule, lequel appareil comportant essentiellement un boîtier (1) pour l'injection, à l'intérieur d'une cuve (5) de mesure, du flux d'échantillon à analyser provenant d'un circuit d'alimentation en amont du boîtier (1) d'injection, laquelle cuve (5) est traversée par un faisceau lumineux recueilli par un capteur (23) et des unités de recueil et de traitement (29, 30, 31) des signaux fournis par le capteur optique (23) et par les électrodes servant à engendrer le champ électrique étant prévues en aval du boîtier (1) d'injection, caractérisé en ce que le flux d'échantillon est manchonné hydrodynamiquement à l'intérieur de la cuve par deux liquides de manchonnage sous pression injectés dans la cuve (5) par deux conduits d'alimentation séparés (14, 19), disposés en amont de la cuve, le second manchonnage enveloppant le premier flux manchonné, et en ce que les deux électrodes pour les mesures de résistivité de la sous-population leucocytaire sont des éléments constitués d'une part, d'une anode formée par l'embout (8) du conduit d'évacuation (6) du flux manchonné de solution sortant de la cuve (5), et d'autre part, d'une cathode formée par une buse interne d'injection (16) de la solution à analyser.

2. Appareil selon la revendication 1, caractérisé en ce qu'une buse externe d'injection (10) se terminant par un orifice calibré (12) est montée à l'intérieur du boîtier (1) et fait communiquer la chambre intérieure (18) de ladite buse avec la cuve (5), pour l'émission du premier liquide de manchonnage et en ce qu'une chambre annulaire (13) qui communique aussi avec la cuve de mesure (5) est ménagée entre une partie conique (11) de ladite buse externe d'injection (10) et une face interne du boîtier d'injection (1), ladite chambre étant alimentée en fluide sous pression par un conduit (14), pour l'émission du second liquide de manchonnage.

3. Appareil selon les revendications 1 et 2, caractérisé en ce qu'une buse interne d'injection (16) est montée à l'intérieur de la buse externe (10) et porte un orifice (21) d'injection de fluide sous pression provenant d'un conduit (20) qui débouche dans une chambre (18) intérieure à ladite buse externe au droit de l'orifice calibré (12).

4. Appareil selon la revendication 1, utilisant une cuve de mesure encadrée par, d'un côté, une lampe et, de l'autre côté, un capteur qui reçoit le faisceau lumineux émis par la lampe et focalisé par des éléments optiques, caractérisé en ce que la cuve de mesure (5) présente un large passage intérieur pour que le flux doublement manchonné reste concentrique et stabilisé pendant tout son trajet dans la cuve.

5. Appareil selon la revendication 1, caractérisé en ce qu'on positionne un ensemble capteur focalisé sur la cuve de mesure (5), à 90° d'un rayon laser le traversant, en vue de réaliser une mesure de cytofluorescence.

6. Appareil selon la revendication 1, caractérisé en ce que le liquide à analyser et les fluides de manchonnage proviennent d'un ensemble de distribution hydraulique (27) alimenté par un échantillon provenant d'une unité (28) de mélange et de chauffage.

7. Appareil selon la revendication 1, caractérisé en ce que les informations provenant des mesures de résistivité sont restituées sur une courbe de répartition des volumes permettant de localiser entre au moins un seuil bas et un seuil haut la population principale de leucocytes, en séparant les stromas et plaquettes ainsi que les particules de très grande taille.

8. Appareil selon la revendication 1, caractérisé en ce que les informations provenant des mesures optiques sont restituées sur une courbe de répartition des absorbances permettant de localiser entre un seuil bas et un seuil haut l'absorbance des leucocytes en éliminant les populations de cellules peu absorbantes ainsi que les populations de cellules à absorbance intense.

9. Appareil selon les revendications 1, 7 et 8, caractérisé en ce que les informations provenant des mesures de résistivité et des mesures optiques sont restituées dans une représentation graphique en matrice, permettant de visualiser des contours représentatifs de la répartition des populations.

## Claims

1. Apparatus for counting and determining at least one leucocytic sub-population using at least in part an analysis and counting method by resistivity based on the principle of a cell passing through an electric field where a constant current is maintained delivering a signal which is interpreted so as to obtain information about the volume of the cell passing through this field and using at least in part an optical method which consists in causing a cell to pass through a light ray and collecting in an optical reader the light absorbed by the cell so as to provide a signal interpreted in order to obtain information about the absorbance of the cell, said apparatus comprising essentially a case (1) for injecting inside a measurement tank (5) the flow of sample to be analysed, which is supplied from a feed circuit upstream of the injection case (1), and said tank (5) is crossed by a light beam collected by a sensor (23) and units (29, 30, 31) for collecting and processing the signals delivered by the optical sensor (23) and by the electrodes used to generate the electric field which are provided upstream of the injection case (1),
characterised in that the sample flow is sleeved hydrodynamically inside the tank by two pressurised sleeving liquids injected into the tank (5) through two separate feed ducts (14, 19) arranged upstream of the tank, the second sleeving enveloping the first sleeved flow, and in that the two electrodes for measurement of the resistivity of the leucocytic sub-population are elements constituted, on the one hand, by an anode formed by the end piece (8) of the discharge duct (6) for the sleeved flow of solution leaving the tank (5), and on the other hand by a cathode formed by an internal nozzle (16) for injection of the solution to be analysed.

2. Apparatus according to claim 1, characterised in that an external injection nozzle (10) ending in a calibrated orifice (12) is mounted inside the case (1) and causes the internal chamber (18) of said nozzle to communicate with the tank (5), for the emission of the first sleeving liquid, and in that an annular chamber (13) which also communicates with the measurement tank (5) is incorporated between a conical part (11) of said external injection nozzle (10) and an internal face of the injection case (1), said chamber being fed with pressurised fluid through a duct (14), for the emission of the second sleeving liquid.

3. Apparatus according to claims 1 and 2, characterised in that an internal injection nozzle (16) is mounted inside the external nozzle (10) and has an orifice (21) for the injection of pressurised fluid coming from a duct (20) which opens into a chamber (18) inside said external nozzle in line with the calibrated orifice (12).

4. Apparatus according to claim 1, using a measurement tank surrounded, on one side, by a lamp and, on the other, by a sensor which receives the light beam emitted by the lamp and focused by optical elements, characterised in that the measurement tank (5) has a wide internal passage so that the doubly sleeved flow remains concentric and stabilised during the whole of its travel through the tank.

5. Apparatus according to claim 1, characterised in that a sensor assembly is positioned, focused on the measurement tank (5), at 90° from a laser ray passing through it, for carrying out a cytofluorescence measurement.

6. Apparatus according to claim 1, characterised in that the liquid to be analysed and the sleeving fluids come from a hydraulic distribution assembly (27) fed by a sample from a mixing and heating unit (28).

7. Apparatus according to claim 1, characterised in that the information coming from the resistivity measurements is restored in the form of a curve of distribution of the volumes for localising, between at least a low threshold and a high threshold, the main leucocyte population, by separating the stromata and platelets as well as the very large sized particles.

8. Apparatus according to claim 1, characterised in that the information coming from the optical measurements is restored by means of an absorbance distribution curve for localising, between a low threshold and a high threshold, the absorbance of the leucocytes by eliminating the cell populations exhibiting little absorbance and the cell populations exhibiting intense absorbance.

9. Apparatus according to claims 1, 7 and 8, characterised in that the information coming from the resistivity measurements and optical measurements is restored in a graphic representation in matrix form, for displaying the contours representative of the distribution of the populations.

## Patentansprüche

1. Gerät zum Zählen und zur Bestimmung mindestens einer leukozytären Unterpopulation, das zumindest teilweise ein Verfahren zur Analyse und zur Zählung durch Widerstand einsetzt, das auf dem Prinzip des Eintritts einer Zelle in ein elektrisches Feld beruht, in dem die Stromstärke konstant gehalten wird, wobei dieser Eintritt ein Signal liefert, das im Hinblick darauf interpretiert wird, eine Information über das Volumen der dieses Feld durchquerenden Zelle zu erhalten, und das zumindest teilweise ein optisches Verfahren einsetzt, das darin besteht, eine Zelle in einen Lichtstrahl eintreten zu lassen und in einem optischen Kollektor das durch die Zelle absorbierte Licht zu sammeln, um ein Signal zu liefern, das im Hinblick darauf interpretiert wird, eine Information über die Absorption der Zelle zu erhalten, wobei dieses Gerät im wesentlichen ein Gehäuse (1) für die Injektion im Inneren einer Küvette (5) zur Messung des zu analysierenden, aus einer Versorgungsleitung stromaufwärts des Injektionsgehäuses (1) stammenden Probenflusses aufweist, wobei die Küvette (5) von einem von einem Meßnehmer (23) aufgefangenen Lichtbündel durchquert wird, und wobei dieses Gerät weiterhin Einheiten zum Empfang und zur Verarbeitung (29, 30, 31) der von dem optischen Meßnehmer (23) und von den Elektroden, die zur Erzeugung des elektrischen Feldes dienen und die stromabwärts des Injektionsgehäuses (1) vorgesehen sind, gelieferten Signale aufweist, dadurch gekennzeichnet, daß der Probenfluß im Inneren der Küvette durch zwei unter Druck in die Küvette (5) über zwei getrennte Versorgungsleitungen (14, 19), die stromaufwärts der Küvette angeordnet sind, injizierte Umhüllungsflüssigkeiten hydrodynamisch umhüllt wird, wobei die zweite Umhüllung den ersten umhüllten Fluß ummantelt, und daß die beiden Elektroden zur Widerstandsmessung der leukozytären Unterpopulation einerseits aus einer Anode, die durch den Ansatz (8) der abführenden Leitung (6) des aus der Küvette (5) austretenden umhüllten Lösungsflusses gebildet ist, und andererseits aus einer Kathode, die durch eine innere Düse (16) zur Injektion der zur analysierenden Lösung gebildet ist, bestehende Elemente sind.

2. Gerät nach Anspruch 1, dadurch gekennzeichnet, daß eine äußere Injektionsdüse (10), die in einer kalibrierten Öffnung (12) mündet, im Inneren des Gehäuses (1) montiert ist und die innere Kammer (18) der Düse mit der Küvette (5) kommunizieren läßt, um die erste Umhüllungsflüssigkeit auszustoßen, und daß eine ringförmige Kammer (13), die auch mit der Meßküvette (5) kommuniziert, zwischen einem konischen Bereich (11) der äußeren Injektionsdüse (10) und einer Innenseite des Injektionsgehäuses (1) untergebracht ist, wobei diese Kammer über eine Leitung (14) mit druckbeaufschlagtem Fluid versorgt wird, um die zweite Umhüllungsflüssigkeit auszustoßen.

3. Gerät nach Anspruch 1 oder 2, dadurch gekennzeichnet, daß eine innere Injektionsdüse (16) im Inneren der äußeren Düse (10) montiert ist und eine Öffnung (21) zur Injektion von druckbeaufschlagtem Fluid aufweist, das aus einer Leitung (20) austritt, die in eine Kammer (18) im Inneren der äußeren Düse gegenüber der kalibrierten Öffnung (12) mündet.

4. Gerät nach Anspruch 1, bei dem eine Meßküvette zum Einsatz kommt, die auf der einen Seite von einer Lampe und auf der anderen Seite von einem Meßnehmer eingerahmt ist, der das von der Lampe emittierte und durch optische Elemente focussierte Lichtbündel empfängt, dadurch gekennzeichnet, daß die Meßküvette (5) zur Erhaltung der Konzentrizität und der Stabilität des doppelt umhüllten Flusses während seiner gesamten Strecke in der Küvette einen weiten inneren Durchgang aufweist.

5. Gerät nach Anspruch 1, dadurch gekennzeichnet, daß zur Durchführung einer Cytofluoreszenz-Messung eine auf die Meßküvette (5) focussierte Meßgruppe in einem 90°-Winkel zu einem die Küvette durchquerenden Laserstrahl angeordnet ist.

6. Gerät nach Anspruch 1, dadurch gekennzeichnet, daß die zu analysierende Flüssigkeit und die Umhüllungsfluide aus einer hydraulischen Verteilergruppe (27) stammen, die mit einer aus einer Einheit (28) zum Mischen und Heizen stammenden Probe versorgt wird.

7. Gerät nach Anspruch 1, dadurch gekennzeichnet, daß die aus den Widerstandsmessungen hervorgegangenen Informationen in einer Volumenverteilungskurve wiedergegeben werden, wodurch zwischen einem unteren und einem oberen Schwellenwert die Hauptpopulation der Leukozyten lokalisiert werden kann, indem die Stromata und Plättchen, wie auch die Partikel von außerordentlicher Größe ausgesondert werden.

8. Gerät nach Anspruch 1, dadurch gekennzeichnet, daß die aus den optischen Messungen gewonnenen Informationen in einer Absorptionsverteilungskurve aufgetragen werden, wodurch zwischen einem unteren und einem oberen Schwellenwert die Absorption der Leukozyten lokalisiert werden kann, indem die Populationen von wenig absorbierenden Zellen, wie auch die Populationen von intensiv absorbierenden Zellen abgeschieden werden.

9. Gerät nach einem der Ansprüche 1, 7 und 8, dadurch gekennzeichnet, daß die aus den Widerstand- und den optischen Messungen hervorgegangenen Informationen in einer graphischen Matrixdarstellung wiedergegeben werden, wodurch die repräsentativen Umrisse der Populationsverteilung sichtbar gemacht werden können.
